(19) Europäisches Patentamt
European Patent Office
Office européen des brevets

(11) Numéro de publication: 0 192 587
A1

(12) # DEMANDE DE BREVET EUROPEEN

(21) Numéro de dépôt: 86420031.6

(22) Date de dépôt: 30.01.86

(51) Int. Cl.⁴: C 07 C 29/136
C 07 C 31/08

(30) Priorité: 08.02.85 FR 8502009

(43) Date de publication de la demande:
27.08.86 Bulletin 86/35

(84) Etats contractants désignés:
AT BE CH DE FR GB IT LI LU NL SE

(71) Demandeur: RHONE-POULENC CHIMIE DE BASE
25, quai Paul Doumer
F-92408 Courbevoie(FR)

(72) Inventeur: Caillod, Jack
31, rue Phanie Leleu
F-95150 Taverny(FR)

(72) Inventeur: Sauvion, Guy-Noel
8, rue du Béarn
F-94550 Chevilly la Rue(FR)

(74) Mandataire: Varnière-Grange, Monique et al,
RHONE-POULENC INTERSERVICES Service Brevets
Chimie Centre de Recherches de Saint-Fons B.P. 62
F-69192 Saint-Fons Cédex(FR)

(54) **Procédé d'hydrogénation de l'acide acétique.**

(57) L'invention concerne un procédé d'hydrogénation de l'acide acétique en phase vapeur pour produire de l'éthanol sous forme libre ou sous la forme combinée de l'acétate d'éthyle.

Il a maintenant été trouvé un procédé d'hydrogénation catalytique de l'acide acétique en phase vapeur, dans des conditions relativement modérées de température et de pression, en vue de produire de l'éthanol et ou de l'acétate d'éthyle, caractérisé en ce que le catalyseur est composé de ruthénium déposé sur un support de silice.

L'invention a également pour objet un procédé de fabrication du catalyseur.



Croydon Printing Company Ltd

0192587

# PROCEDE D'HYDROGENATION DE L'ACIDE ACETIQUE

La présente invention concerne un procédé d'hydrogénation de l'acide acétique.

L'invention a plus particulièrement pour objet un procédé d'hydrogénation de l'acide acétique en phase vapeur pour produire de l'éthanol sous forme libre ou sous la forme combinée de l'acétate d'éthyle.

L'hydrogénation de l'acide acétique répresente une voie intéressante de production de l'éthanol mais le développement à l'échelle industrielle de cette technique a été freiné jusqu'alors par les inconvénients des catalyseurs connus.

De nombreux travaux antérieurs concernant l'hydrogénation en phase liquide d'acides gras en esters ou alcools lourds ont mis en évidence l'intérêt de catalyseurs à base d'oxydes mixtes de cuivre et de chrome, éventuellement dopés par de l'oxyde de zinc (par exemple). Toutefois ces systèmes ne sont efficaces que dans des conditions extrêmement sévères de pression et de température puisqu'il convient généralemennt de travailler sous des pressions supérieures à 200 atmosphères et à des températures situées entre 250 et 350 °C.

On a également proposé d'hydrogéner l'acide acétique en phase liquide en présence de dioxyde de ruthénium ou de ruthénium sur du charbon (cf US 2 607 807). Toutefois l'intérêt réel de ces techniques est largement compromis par les hautes pressions (supérieures à 200 atm dans le cadre de la technique décrite dans l'US 2 607 807) qui sont requises ainsi que par le manque d'efficacité des catalyseurs en cause.

A cela s'ajoute le manque de sélectivité observé lors de la mise en oeuvre de ce type de technique qui grève lourdement l'économie globale de ces procédés.

Plus récemment, il a été proposé de conduire l'hydrogénation des acides carboxyliques en phase vapeur sous des pressions et températures relativement modérées, éventuellement en présence d'un excès de vapeur d'eau, sur un catalyseur comprenant des oxydes mixtes du ruthénium et d'au moins l'un des métaux choisis parmi le cobalt et le nickel (cf. US 4 398 039). Toutefois, malgré le recours à des catalyseurs

relativement compliqués à préparer et dont la stabilité peut être mise en doute, cette technique est peu efficace tout en nécessitant un très grand excès d'hydrogène par rapport au substrat à hydrogéner.

Il apparaît donc clairement qu'il serait très souhaitable de disposer d'un procédé d'hydrogénation de l'acide acétique, capable de fonctionner dans des conditions relativement modérées de pression, qui permettrait d'accéder avec une bonne sélectivité à l'éthanol et/ou à l'acétate d'éthyle sans présenter pour autant les divers inconvénients des procédes antérieurs.

Il a maintenant été trouvé et c'est ce qui constitue l'objet essentiel de la présente invention un procédé d'hydrogénation catalytique de l'acide acétique en phase vapeur, dans des conditions relativement modérées de température et de pression, en vue de produire de l'éthanol et/ou de l'acétate d'éthyle, caractérisé en ce que le catalyseur est composé de ruthénium déposé sur un support de silice.

La présente invention a également pour objet un procédé de préparation d'un tel catalyseur.

Le catalyseur en cause comporte donc du ruthénium qui peut être déposé sur un support de silice par tout moyen approprié. En particulier ce catalyseur pourra être préparé par imprégnation du support de silice avec une solution aqueuse d'un précurseur du ruthénium métallique, soluble dans l'eau. A titre d'exemple de tels précurseurs on peut citer :

- le trichlorure de ruthénium $RuCl_3$, 3 $H_2O$
- le tribromure de ruthénium
- le pentachlororuthénate d'ammonium :
  $(RuCl_5, 4H_2O) (NH_4)_2$
- le chlorure de ruthénium chlorohexammine :
  $[ RuCl(NH_3)_6 ] Cl_3$.

On recourra avantageusement au chlorure de ruthénium notamment en raison de sa grande plus disponibilité.

Le support de silice, second constituant fondamental du catalyseur utilisé dans la mise en oeuvre du procédé d'hydrogénation selon l'invention, est avantageusement choisi parmi les silices présentant les caractéristiques suivantes :

- le volume poreux total, déterminé par porosimètre à mercure

est compris entre 0,2 et 1,2 $cm^3.g^{-1}$ et, de préférence entre 0,4 et 0,8 $cm^3.g^{-1}$,

- la surface spécifique, mesurée selon la méthode B.E.T. est comprise entre 100 et 500 $m^2.g^{-1}$ et, de préférence entre 200 et 400 $m^2.g^{-1}$,

- la densité de grain est comprise entre 0,50 et 1,50 et, de préférence entre 0,65 et 0,95,

- la granulomètrie est comprise entre 1 et 5 mm et, de préférence entre 2 et 4 mm.

On utilisera avantageusement des silices dont les caractéristiques sont toutes choisies dans les gammes de valeurs indiquées ci-avant comme préférentielles.

Dans l'ensemble (ruthénium + support de silice) le ruthénium représente en général de 0,5 à 5 % en poids (exprimé en métal).

Le catalyseur peut avantageusement être préparé par imprégnation du support de silice choisi, à la température ambiante, au drageoir par exemple, par un volume de solution aqueuse contenant la quantité de précurseur métallique désirée, sensiblement égale au volume poreux du support.

L'imprégnation est suivie d'une part d'un séchage, effectué à une température comprise entre environ 110 et 170°C pendant une durée minimale de 2 heures sans qu'il soit utile de dépasser 24 heures, et d'autre part, d'une calcination à une température de 300 à 500°C dont la durée est généralement comprise entre 1 et 4 heures.

Avant son utilisation dans le procédé d'hydrogénation, objet de la présente invention, le catalyseur doit subir un traitement de réduction. Ce traitement est avantageusement effectué à une température comprise entre 250 et 400°C sous courant d'hydrogène gazeux dont la vitesse spatiale est comprise entre 1500 et 6000 $h^{-1}$.

Bien évidemment, ce traitement peut aussi être réalisé par d'autres techniques, par exemple en recourant à un agent réducteur organique tel l'aldéhyde formique ou l'hydrazine.

Pour une bonne mise en oeuvre du procédé d'hydrogénation selon l'invention, on utilisera une quantité de catalyseur solide (par rapport à l'acide acétique à hydrogéner) de telle sorte que l'on puisse

hydrogéner par heure de fonctionnement de 0,5 à 3 parties d'acide acétique par partie de catalyseur.

Selon la présente invention on met donc à réagir en phase gazeuse de l'acide acétique et de l'hydrogène en présence d'un catalyseur, décrit ci-avant.

La température de réaction est en général comprise entre 200 et 500°C et, plus particulièrement entre 250 et 350°C.

La pression à la température choisie pour la réaction est en général comprise entre 10 et 200 atm, tout en étant compatible avec le maintien des réactifs et produits en phase gazeuse. Cette pression est avantageusement comprise entre 30 et 100 atm.

La réaction d'hydrogénation en cause dans le procédé selon l'invention peut être représentée comme suit :

$$CH_3COOH + 2\ H_2 \longrightarrow CH_3CH_2OH + H_2O$$

Le rapport molaire de l'hydrogène à l'acide acétique est au moins égal à celui indiqué par la stoéchiométrie de la réaction et, en général, on opère avec un excès d'hydrogène par rapport à cette stoéchiométrie. Aucun avantage particulier n'est observé lorsque ce rapport molaire excède 50. De manière avantageuse on fixera ce rapport molaire à une valeur comprise entre 10 et 20.

Si l'introduction de diluants inertes tel $CO_2$ s'avère possible en principe, elle ne présente cependant guère d'intérêt en pratique dans la mesure où elle conduirait à une baisse de la presssion partielle des réactifs et par là, à une diminution des performances du système.

On notera toutefois que l'introduction d'eau, qui de toutes façons est produite par la réaction, peut être intéressante puisqu'elle permet d'orienter la réaction au profit de l'éthanol en déplaçant l'équilibre d'estérification :

$$CH_3COOH + CH_3CH_2OH \rightleftarrows CH_3COOC_2H_5 + H_2O$$

En général, la vitesse spatiale gazeuse (rapport du volume gazeux des réactifs, mesuré dans les conditions normales de température et de pression, au volume de catalyseur par unité de temps) est comprise entre 100 et 10 000 $h^{-1}$ et elle est, de préférence inférieure à 6000 $h^{-1}$.

Les exemples ci-après illustrent l'invention.

## EXEMPLES

## PREPARATION ET CARACTERISATION DES CATALYSEURS

### EXEMPLE 1

Cet exemple indique les caractéristiques chimiques et physiques d'un catalyseur massique à base de chromite de cuivre, représentatif des catalyseurs traditionnellement utilisés dans l'art antérieur.

La composition de ce catalyseur est la suivante, les pourcentages indiqués étant des pourcentages en poids :

| | | |
|---|---|---|
| - oxyde de chrome | $Cr_2O_3$ | 44 % |
| - oxyde de cuivre | CuO | 36 % |
| - oxyde de baryum | BaO | 10 % |
| - silice | $SiO_2$ | 10 % |

Ce catalyseur se présente sous la forme d'extrudés dont le diamètre est de 3 mm pour une longueur de 3 mm.

La densité de grain de ce catalyseur mesurée par picnomètrie au mercure est de 2,40. Son volume poreux total est de 0,10 $cm^3.g^{-1}$.

Sa surface spécifique B.E.T. est de 80 $m^2.g^{-1}$.

### EXEMPLE 2

Cet exemple illustre la préparation d'un catalyseur composé de rhodium déposé sur un support de silice, qui n'entre pas dans le cadre de la présente invention.

On imprègne de la silice dont les caractéristiques sont les suivantes par une solution aqueuse de nitrate de rhodium

$Rh(NO_3)_3.2\ H_2O$

Le support de silice se présente sous forme de billes concassées dont seule la fraction dont la dimension est comprise entre 2 et 3 mm est conservée. La densité de remplissage tassé mesurée sur une éprouvette de 300 $cm^3$ dont le tassement a été assuré par un vibreur (50 périodes), est de 0,49. Le volume poreux à l'eau déterminé pour effectuer une

imprégnation dite "à sec" du support (et légèrement inférieur au volume poreux total) est de 0,56 $cm^3.g^{-1}$. La surface spécifique B.E.T. est de 240 $m^2.g^{-1}$.

On imprègne, au drageoir, à la température ambiante, en deux étapes, 100 $cm^3$ de support décrit ci-avant, à chaque fois par 55 $cm^3$ d'une solution aqueuse contenant 1,43 g de nitrate de rhodium.

Le catalyseur ainsi préparé est alors séché pendant 16 heures à 150°C, puis calciné pendant 2 heures à 450°C. Il présente alors les caractéristiques suivantes :

- la teneur en rhodium est de 1 % en poids,
- la densité de grain, mesurée comme indiquée à l'exemple 1, est de 0,94,
- le volume poreux à l'eau est de 0,51 $cm^3.g^{-1}$,
- la surface spécifique B.E.T. est de 220 $m^2.g^{-1}$.

EXEMPLE 3

Cet exemple illustre la préparation d'un catalyseur comprenant des oxydes mixtes du ruthénium et du cobalt, répondant à la formule : Ru Co Ni $Zn_{0.4}$ $O_x$ indiquée à l'exemple 17 du brevet américain n° 4 398 039. Ce catalyseur n'entre pas dans le cadre de la présente invention.

A 250 ml d'eau on ajoute :

- 3,8 g de chlorure de ruthénium $Ru\ Cl_3$, 3 $H_2O$,
- 3,6 g de chlorure cobalteux $Co\ Cl_2$, 6 $H_2O$,
- 3,2 g de chlorure palladium $Pd\ Cl_2$, 2 $H_2O$,
- 8,1 g de chlorure de zinc $Zn\ Cl_2$,

soit respectivement 0,015 mole de ruthénium, de cobalt et de palladium et 0,006 mole de zinc.

On soumet l'ensemble à agitation pendant 30 minutes. Le pH de la solution est alors amené à 8,3 par ajoût de soude. On porte alors l'ensemble sous agitation, à une température de l'ordre de 90 °C pendant 30 minutes, puis on ajuste le pH à 7,5.

On filtre et on lave le gâteau, le tout à deux reprises, puis on le sèche pendant 16 heures à 125°C. Le catalyseur est alors calciné pendant 3 heures à 350°C avant d'être concassé et tamisé, seule la

fraction dont la granolumétrie est de 3 ± 0,5 mm est retenue.

EXEMPLE 4

Cet exemple illustre la préparation d'un catalyseur selon l'invention.

La silice utilisée comme support dudit catalyseur est identique à celle utilisée dans la préparation du catalyseur décrite dans l'exemple 2.

On imprègne 100 $cm^3$ de cette silice, au drageoir , à la température ambiante, par 27,4 $cm^3$ d'une solution aqueuse contenant 1 g de chlorure de ruthénium trihydraté.

Le catalyseur ainsi préparé est alors séché pendant 16 heures à 120°C.

Il présente alors les caractéristiques suivantes :

- Sa teneur en ruthénium est de 0,8 % en poids,
- Sa densité de grain mesurée comme indiqué précédemment est de 0,95,
- Son volume poreux à l'eau est de à 0,51 $cm^3.g^{-1}$,
- Sa surface spécifique B.E.T. est de 210 $m^2.g^{-1}$.

COMPARAISON DES DIFFERENTS CATALYSEURS DANS LA REACTION D'HYDROGENATION DE L'ACIDE ACETIQUE, EN PHASE VAPEUR

EXEMPLE 5

On a réalisé une série d'essais sur un mélange de réactifs comprenant 94 % molaire d'hydrogène et 6 % d'acide acétique, ($H_2/CH_3COOH$ = 15,66 Molaire) dans le but de comparer les performances des divers catalyseurs décrits ci-avant en termes d'activité et de sélectivité dans la synthèse d'éthanol et d'acétate d'éthyle.

Dans tous les cas la réaction est effectuée en présence de 30 $cm^3$ de catalyseur, à la vitesse spatiale de 5300 $h^{-1}$, à la température de 300°C et sous une pression de 60 bars.

Auparavant, le catalyseur subit un traitement de réduction à 300°C sous un courant de 100 $l.h^{-1}$ d'hydrogène pendant 3 heures.

Après séparation des produits par un condenseur :

- les gaz permanents sont analysés par chromatographie en ligne à détection par catharométrie
- les liquides sont récupérés toutes les deux heures et analysés par chromatagraphie en phase gazeuse à programmation de température et détection par ionisation de flamme.

Ces analyses permettent de déterminer :

- le taux de conversion de l'acide acétique (%), défini par le rapport :

$$t_{AcOH} = \frac{\text{moles de } CH_3COOH \text{ consommées}}{\text{moles de } CH_3COOH \text{ introduites}} \times 100$$

- la sélectivité en acétate d'éthyle (%), définie par le rapport :

$$S_{AcOET} = \frac{\text{moles de } CH_3COOC_2H_5 \text{ formées}}{\text{moles de } CH_3COOH \text{ consommées}} \times 2 \times 100$$

- la sélectivité en éthanol (%), définie par le rapport :

$$S_{EtOH} = \frac{\text{moles de } CH_3CH_2OH \text{ formées}}{\text{moles de } CH_3COOH \text{ consommées}} \times 100$$

Dans le tableau I, figurent les résultats obtenus sur les 4 catalyseurs décrits précédemment respectivement après 3 heures et 50 heures de fonctionnement dans les conditions qui ont été définies, la productivité indiquée par Pr étant le nombre de grammes d'acétate d'éthyle et d'éthanol obtenu par heure et par litre de catalyseur, après 50 heures de fonctionnement.

TABLEAU I

| Catalyseur | $t_{AcOH}$ (%) 3 h | $t_{AcOH}$ (%) 50 h | $S_{AcOEt}$ (%) 3 h | $S_{AcOEt}$ (%) 50 h | $S_{EtOH}$ (%) 3 h | $S_{EtOH}$ (%) 50 h | Pr |
|---|---|---|---|---|---|---|---|
| Exemple n°1 Chromite de cuivre | 8,4 | 6,1 | 48,7 | 49,5 | 51,3 | 50,5 | 58 |
| Exemple n°2 $Rh/SiO_2$ | 4,1 | 2,3 | 77,6 | 80,3 | 22,4 | 19,7 | 22 |
| Exemple n°3 $RuCoPdZn_{0,4}O_x$ | 7,2 | 6,3 | 63,5 | 67,4 | 36,5 | 32,6 | 60 |
| Exemple n°4 $Ru/SiO_2$ | 55,2 | 54,5 | 47,2 | 48,9 | 52,8 | 51,1 | 515 |

COMPORTEMENT D'UN CATALYSEUR SELON L'INVENTION DANS DIVERSES CONDITIONS OPERATOIRES

EXEMPLE 6

Le catalyseur dont la préparation a été décrite à l'exemple 4 et qui a été réduit comme indiqué à l'exemple 5 est testé à diverses reprises, selon le mode opératoire décrit à l'exemple 5, la vitesse spatiale étant portée successivement aux valeurs de :

11 000 $h^{-1}$ ( essai 6 a)
9 000 $h^{-1}$ ( essai 6 b)
6 000 $h^{-1}$ ( essai 6 c)
4 000 $h^{-1}$ ( essai 6 d)

Les résultats obtenus dans chaque cas figurent dans le tableau II ci-après dans lequel les conventions utilisées sont les mêmes que précédemment.

TABLEAU II

| Ref | Vitesse spatiale ($h^{-1}$) | $t_{AcOH}$ (%) | $S_{AcOEt}$ (%) | $S_{EtOH}$ (%) | Pr |
|---|---|---|---|---|---|
| 6a | 11 000 | 37,6 | 42,4 | 57,6 | 737 |
| 6b | 9 000 | 42,1 | 45,3 | 54,7 | 675 |
| 6c | 6 000 | 52,3 | 49,8 | 50,2 | 560 |
| 6d | 4 000 | 61,6 | 59,2 | 40,8 | 439 |

EXEMPLE 7

Le catalyseur dont la préparation a été décrite à l'exemple 4 et qui a été réduit comme indiqué à l'exemple 5 est testé à diverses reprises, selon le mode opératoire décrit à l'exemple 5, le rapport molaire $H_2/CH_3COOH$ étant porté successivement au valeurs de :

5 (essai 7 a)
10 (essai 7 b)
50 (essai 7 c)

Les résultats obtenus dans chaque cas figurent dans le tableau III ci-après dans lequel les conventions utilisées sont les mêmes que précédemment.

TABLEAU III

| Ref | Rapport Molaire $H_2/CH_3COOH$ | $t_{AcOH}$ (%) | $S_{AcOEt}$ (%) | $S_{EtOH}$ (%) | Pr |
|---|---|---|---|---|---|
| 7a | 5 | 15,3 | 67,6 | 32,4 | 463 |
| 7b | 10 | 32,5 | 58,7 | 41,3 | 491 |
| 7c | 50 | 77,1 | 50,6 | 49,4 | 233 |

EXEMPLE 8

On reproduit l'exemple 5 avec le catalyseur dont la préparation est décrite à l'exemple 4 en ajoutant cette fois-ci de l'acide acétique en présence d'eau dans un rapport molaire $H_2O/CH_3COOH$ de 5. La composition molaire du gaz à l'entrée est donc la suivante :

- $CH_3COOH$ : 4,5 %
- $H_2O$ : 22,5 %
- $H_2$ : 73,0 %

Les résultats ainsi obtenus sont les suivants :

- taux de conversion de $CH_3COOH$ : 22,6 %
- sélectivité en ETOH : 91,2 %
- sélectivité en AcOET : 8,8 %

Ce qui correspond à une productivité de 160g d'(AcOET + ETOH) par heure et par litre de catalyseur.

## REVENDICATIONS

1°) Procédé d'hydrogénation catalytique de l'acide acétique en phase vapeur dans des conditions relativement modérées de température et de pression, en vue de produire de l'éthanol et/ou de l'acétate d'éthyle caractérisé en ce que le catalyseur est composé de ruthénium déposé sur un support de silice.

2°) Procédé selon la revendication 1, caractérisé en ce que le support est une silice comportant les caractéristiques suivantes :

- volume poreux total compris entre 0,2 et 1,2 $cm^3.g^{-1}$,
- surface spécifique comprise entre 100 et 500 $m^2.g^{-1}$,
- densité de grain comprise entre 0,5 et 1,50,
- granulométrie comprise entre 1 et 5 mm.

3°) Procédé selon la revendication 1 ou 2, caractérisé en ce que le support est une silice comportant les caractéristiques suivantes :

- volume poreux total compris entre 0,4 et 0,8 $cm^3.g^{-1}$,
- surface spécifique comprise entre 200 et 400 $m^2.g^{-1}$,
- densité de grain comprise entre 0,65 et 0,95,
- granulométrie comprise entre 2 et 4 mm.

4°) Procédé selon l'une quelconque des revendications précédentes, caractérisé en ce que le ruthénium représente de 0,5 à 5 % en poids du catalyseur.

5°) Procédé selon l'une quelconque des revendications précédentes, caractérisé en ce que la température de réaction est comprise entre 200 et 500°C.

6°) Procédé selon l'une quelconque des revendications précédentes, caractérisé en ce que la pression est comprise entre 10 et 200 atmosphères.

7°) Procédé selon l'une quelconque des revendications précédentes, caractérisé en ce que le rapport molaire $H_2/CH_3COOH$ est compris entre 2 et 50.

8°) Procédé selon l'une quelconque des revendications précédentes, caractérisé en ce que la vitesse spatiale gazeuse est comprise entre 100 et 10 000 $h^{-1}$.

9°) Procédé selon l'une quelconque des revendications précédentes, caractérisé en ce que le catalyseur subit une réduction au moins partielle, avant son utilisation.

10°) Procédé de préparation d'un catalyseur pour la mise en oeuvre du procédé selon l'une quelconque des revendications précédentes caractérisé par la succession d'étapes suivantes :

a) on imprègne une silice comportant les caractéristiques suivantes :)

- volume poreux total compris entre 0,2 et 1,2 $cm^3.g^{-1}$,
- surface spécifique comprise entre 100 et 500 $m^2.g^{-1}$,
- densité de grain comprise entre 0,5 et 1,50,
- granulométrie comprise entre 1 et 5 mm.

au moyen d'une solution aqueuse d'un précurseur du ruthénium soluble dans l'eau,

b) on sèche l'ensemble,

c) on le calcine, le cas échéant, et

d) on réduit le ruthénium à l'état de métal au moyen d'hydrogène.

0192587

Office européen des brevets

# RAPPORT DE RECHERCHE EUROPEENNE

Numero de la demande

EP 86 42 0031

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl 4) |
|---|---|---|---|
| D,A | US-A-2 607 807 (TH.A. FORD) * Colonne 1, lignes 1-4, 37-50; colonne 3, lignes 33-57; colonne 4, lignes 16-45 * --- | 1 | C 07 C 29/136<br>C 07 C 31/08 |
| A | FR-A-2 436 130 (MONTEDISON) * Page 12, revendications 1,3,5; page 14, revendication 16 * ----- | 1 | |
| | | | DOMAINES TECHNIQUES RECHERCHES (Int Cl 4) |
| | | | C 07 C 29/00 |

Le présent rapport de recherche a été établi pour toutes les revendications

| Lieu de la recherche | Date d achevement de la recherche | Examinateur |
|---|---|---|
| LA HAYE | 13-05-1986 | KINZINGER J.M. |

CATEGORIE DES DOCUMENTS CITES

X particulièrement pertinent à lui seul
Y particulièrement pertinent en combinaison avec un autre document de la même categorie
A arrière-plan technologique
O divulgation non-écrite
P document intercalaire

T theorie ou principe à la base de l invention
E document de brevet antérieur mais publie a la date de depôt ou apres cette date
D cité dans la demande
L cité pour d autres raisons

& membre de la même famille document correspondant

OEB Form 1503 03 82